# EUROPEAN PATENT APPLICATION

(11) **EP 1 308 144 A1**
(43) Date of publication of application: **07.05.2003**
(21) Application number: 02022634.6
(22) Date of filing: 09.10.2002
(51) Int. Cl.: A61F 5/01

(54) **Modular joint brace**

(30) Priority: 10.10.2001 IT VR20010104
(71) Applicant: F.G.P. SRL, 37062 Dossobuono (IT)
(72) Inventor: Turrini, Alberto, 37060 Castel D'Azzano (VR) (IT); Ferrigolo, Moreno, 37062 Dossobuono (VR) (IT)
(74) Representative: Petraz, Gilberto Luigi

(57) **Abstract**

A modular knee brace (10) comprises a central frame (11) equipped with articulated joints (15), means of constraint of this frame (11) to the femur and to the tibia, a peripheral frame (12, 13) presenting at least one upper arch (24), when in use positioned above the knee and enveloping the front part of the femur, and a lower arch (25) opposite the previous one with respect to the knee. At least one of the arches (24, 25) is constrained to form a single body with the peripheral frames (12, 13) by appropriate clamping means, being at the same time removable and interchangeable.

## Description

### TECHNICAL FIELD

This invention concerns a modular knee brace which can be applied during sports activities and post-operative rehabilitation.

A knee brace of this kind is normally applied to achieve control of translation, hyperextension and rotation of the tibia.

This invention can be applied in the medical industry with particular reference to manufacturers of prostheses and braces.

### BACKGROUND ART

It is a known fact that a subject presenting with an injured or hyperextended cruciate ligament as a result of a previous surgical operation needs to use a knee brace to ensure the function of articulation constraint between the femur and the tibia, supporting strain which would otherwise be harmful to the ligament.

The knee brace usually consists of a rigid and enveloping frame in order to provide an adequate harness that prevents the onset of strain on the ligament during locomotion of the injured and/or convalescent subject.

The frame comprises means of constraint acting on the femur and the tibia at points proximal to the knee and a structure connecting these means with an articulated joint at the knee.

To ensure adequate freedom of movement of the limb, the frame develops almost exclusively at the sides of the knee in order to allow correct reciprocal oscillation between the femur and the tibia.

The means of constraint usually consist of metal arches which envelop both the femur and the fibula of the injured subject.

One drawback is represented by the fact that since these arches have to closely follow the user's limb, their size has to be appropriate to the subject using the brace.

Various sizes of knee brace are therefore necessary in order to satisfy all possible requirements.

This problem is felt to a greater extent in the case of subjects with an excessive accumulation of adipose tissue on the thighs or more in general in situations in which the femoral or tibial part present dimensions that are not comparable with standard sizes.

Considerable difficulties are often encountered by such subjects when using traditional type knee braces, whose upper and lower arches have standardised dimensions, as they are unable to adapt the knee brace to their physical shape and size.

### DESCRIPTION OF THE INVENTION

This invention aims to provide a modular knee brace which, by substituting interchangeable parts, can satisfy the requirements of different sizes.

This invention also aims to provide a modular knee brace which is easy to produce so as to be advantageous from an economic point of view.

An additional aim of this invention is to provide a safe and reliable knee brace in order not to injure the person using it.

This is achieved by means of a modular knee brace with the features described in the main claim.

The dependent claims describe advantageous forms of embodiment of the invention.

The knee brace according to the present invention comprises a central frame equipped with articulated joints, means of constraint for this frame on the upper and lower part of the leg, a peripheral frame presenting at least one upper arch, which when applied is above the knee and envelops the front part of the femur, and a lower arch opposite to the previous one with respect to the knee, it being possible to fix the upper arch to form a single body with peripheral frame, while still being removable and interchangeable.

According to a particular form of embodiment of the invention, the means of fixing of the upper arch and/or the lower arch to the peripheral frame consist of a coupling system comprising plates fixed to form a fork at the ends of the side uprights whose inner part houses the end section of the respective arch.

The plates can be fixed to the uprights by appropriate means such as for example by rivets.

According to an favourable form of embodiment of the invention the arch is fixed to the uprights by the application of a screw which goes through both the plates and the end part of the upper arch and goes through the holes drilled in the frame and in the respective arch.

Maintaining the same dimensions of the central frame and the peripheral frame it is therefore possible to use arches of different measurements in order to adapt one knee brace to different personal sizes merely by interchanging the arches.

The central frame can be made from self-lubricating composite plastic material with a low friction coefficient to ensure good mobility of the articulated joints.

The peripheral frame and the arches can be made from lightweight metal material to ensure good mechanical resistance and consequently greater reliability of the modular knee brace.

### DESCRIPTION OF THE DRAWINGS

Other features and advantages of the invention will become apparent from the following description of an embodiment of the invention, provided purely as a non-restricting example, with reference to the accompanying drawings, in which:

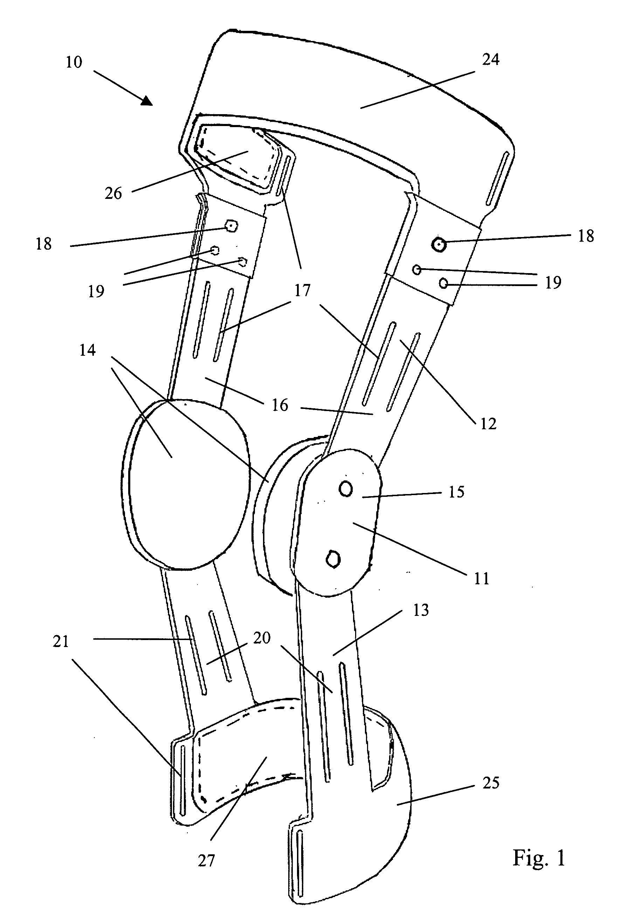

- figure 1 shows a perspective side view, slightly from above, of a knee brace according to the present invention;
- figure 2 shows an enlarged diagrammatic front elevation of the coupling of an arch to the central frame; and
- figure 3 shows a diagrammatic side view of the coupling represented in figure 2.

### DESCRIPTION OF ONE EMBODIMENT

In the figures, the reference number 10 generally indicates a knee brace normally used in the field of injury prevention, post-operative rehabilitation or following knee surgery.

The knee brace 10 comprises a central frame 11, an upper peripheral frame 12 and a lower peripheral frame 13 designed to harness the limb of the user close to the knee.

The central frame 11 presents a pair of plates 14, when in use resting on the condylar parts of the knee, each plate being designed to support an articulated joint 15 which is hinged to it.

Each plate 14 is integral with the peripheral femoral frame 12 while each articulated joint 15 is hinged both to the peripheral tibial frame 13 and to the respective plate 14 to allow controlled angular excursion.

The upper peripheral frame 12 consists of a pair of uprights 16, the central part of each one presenting at least one lightening slot 17 which may also house means of fixing, for example straps, the brace to the user's femur.

In accordance with the invention, the longitudinal end, distal from the articulated joint 15, of each upright 16 is equipped with holes designed to house appropriate mechanical means of clamping such as for example, according to an advantageous embodiment, rivets 19.

The lower peripheral frame 13 comprises a pair of lower uprights 20, which are also equipped with lightening slots 21.

The side uprights 16 of the upper peripheral frame 12 are designed to support a rigid upper arch 24, while the lower uprights 20 of the lower peripheral frame 13 are designed to support a respective lower arch 25 positioned at the opposite longitudinal end of the brace 10.

The arches 24, 25 project from opposite ends of the respective side uprights 16, 20 with, when in use, the upper arch 24 facing the front part and the lower arch 25 facing the rear part of the user's leg.

The inner surface of each arch 24, 25 can be fitted with a respective element made from relatively soft material, such as fabric 26, 27, designed to act as a pressure reducer able to distribute the clamping force over its surface in order to ease the grip of the arches 24, 25 during use of the knee brace 10.

According to a particular form of embodiment of the present invention, the knee brace 10 can be equipped with additional means of fixing to the user's limb, such as for example straps with adjustable tightening for example by means of Velcro strips (not shown in the drawings).

These additional fixing means are designed to ensure a stable and reliable fixing of the brace 10 to the limb.

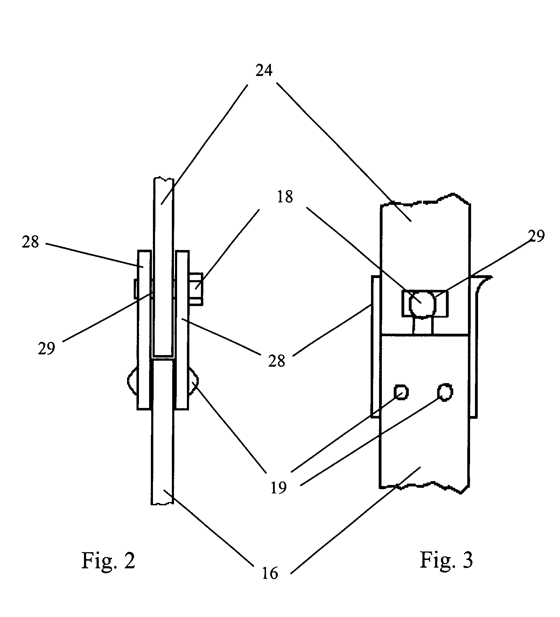

As can be seen in figures 2 and 3, the upper arch 24 according to this invention is interchangeable, despite being fixed to form a single body with the side uprights 16.

The free end of each upright is constrained to a fork comprising a pair of plates 28 equipped with a hole 29 for the insertion of a retaining element such as, for example, a screw 18.

The plates 28 can be fixed to the relative upright 16 by any appropriate mechanical means, for example according to the present form of embodiment by means of rivets 19.

To disengage the arch 24 it is sufficient to unscrew the screw 19 and extract the arch from the forks.

According to a particularly advantageous variation of the present invention not shown in the drawings, the lower arch 25 can also be fixed to form a single body with the side uprights 20 of the lower peripheral frame 13, with the possibility of being removed and replaced if necessary.

The peripheral frames 12, 13 can be made from metal, for example aluminium, the arches 24 and 25 can be made from highly resistant composite plastic material or from metal, for example aluminium, while the central frame 11 can be made from self-lubricating composite plastic material with a low friction coefficient to ensure good mobility of the articulated joints 15.

It is obvious from the above description that the invention fulfils the predetermined aims, since it provides a knee brace that can be fully personalised according to the physical structure of the subject wearing it.

This is particularly advantageous for example in the case of elderly subjects, in many of whom the upper part of the leg is much larger than the lower part.

Thanks to this invention, these subjects who normally have difficulty in adapting to traditional type knee braces, with standard size upper and lower arches, are able to adapt a knee brace perfectly to their physical shape and size.

The invention is described with reference to a preferred embodiment.

It is nevertheless clear that the invention is susceptible to numerous variations within the framework of technical equivalents.

By way of example, it is clear that the principles of the invention can be easily adapted, without the need for any inventive activity, to any other type of knee brace, for example to braces for the treatment of varus/valgus deformity of the knee.

## Claims

1. A modular knee brace (10) comprising a central frame (11) equipped with articulated joints (15), means of constraint of this frame (11) to the femur and to the tibia, a peripheral frame (12, 13) presenting at least one upper arch (24), when in use positioned above the knee and enveloping the front part of the femur, and a lower arch (25) opposite the previous one with respect to the knee, **characterised in that** at least one of the arches (24, 25) is constrained to form a single body with the peripheral frames (12, 13) by appropriate clamping means, being at the same time removable and interchangeable.

2. A knee brace (10) according to claim 1, **characterised in that** the clamping means of the upper arch (24) and/or of the lower arch (25) to the peripheral frames (12, 13) consist of a housing system for the ends of the upper and/or lower arch in which at least one restraining element (18) can be inserted.

3. A knee brace (10) according to any of the preceding claims, **characterised in that** the restraining element (18) consists of a screw.

4. A knee brace (10) according to any of the preceding claims, **characterised in that** the housing at the ends of the side uprights (16, 20) consists of a fork formed by a pair of drilled plates (28) fixed to the uprights.

5. A knee brace (10) according to claim 4, **characterised in that** the drilled plates are fixed to the side uprights (16, 20) by means of rivets.

6. A knee brace (10) according to any of the preceding claims, **characterised in that** the central frame (11) presents two plates (14), which when in use rest against the condylar parts of the knee (14) and which are designed to support the respective articulated joint (15).
